(19) Europäisches Patentamt / European Patent Office / Office européen des brevets

(11) **EP 2 313 868 B1**

(12) **EUROPEAN PATENT SPECIFICATION**

(45) Date of publication and mention of the grant of the patent:
**16.03.2016 Bulletin 2016/11**

(51) Int Cl.:
*A61F 2/76* *(2006.01)*        *G05B 19/4099* *(2006.01)*
*G06T 17/00* *(2006.01)*        *G06T 19/20* *(2011.01)*
*A61F 2/50* *(2006.01)*

(21) Application number: **08783275.4**

(22) Date of filing: **18.07.2008**

(86) International application number:
**PCT/CA2008/001362**

(87) International publication number:
**WO 2010/006405 (21.01.2010 Gazette 2010/03)**

(54) **METHOD, APPARATUS, SIGNALS AND MEDIA FOR PRODUCING A COMPUTER REPRESENTATION OF A THREE-DIMENSIONAL SURFACE OF AN APPLIANCE FOR A LIVING BODY**

VERFAHREN, VORRICHTUNG, SIGNALE UND MEDIEN ZUM ERSTELLEN EINER COMPUTERREPRÄSENTATION EINER DREIDIMENSIONALEN OBERFLÄCHE EINES GERÄTS FÜR EINEN LEBENDEN KÖRPER

PROCÉDÉ, APPAREIL, SIGNAUX ET SUPPORTS POUR PRODUIRE UNE REPRÉSENTATION PAR ORDINATEUR D'UNE SURFACE TRIDIMENSIONNELLE D'UN APPAREIL POUR UN CORPS VIVANT

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**27.04.2011 Bulletin 2011/17**

(73) Proprietor: **Vorum Research Corporation Vancouver, BC V6P 6T3 (CA)**

(72) Inventor: **SABISTON, Robert Malcolm Vancouver,
BC V5P 4X1 (CA)**

(74) Representative: **Awapatent AB**
**P.O. Box 11394**
**404 28 Göteborg (SE)**

(56) References cited:
WO-A2-02/34157        CA-A1- 2 405 738
JP-A- 2003 299 679        US-A1- 2006 094 951

• **JENSEN NIELSEN, K.: 'Bio-Surfaces and Geometric References for a Standardized Biomechanical Design Methodology for Mass Customization' PHD THESIS, BRIGHAM YOUNG UNIVERSITY, [Online] April 2008, pages 1 - 136, XP008142019 Retrieved from the Internet: <URL:http://contentdm.lib.byu.edu/ETD/image /etd2251.pdf>**

**Description**

**BACKGROUND OF THE INVENTION**

**1. Field of Invention**

This invention relates generally to three-dimensional shape representations and more particularly to producing a computer representation of a three-dimensional surface of an appliance for a living body

**2. Description of Related Art**

Prostheses, orthoses, and other support appliances are commonly produced from three-dimensional representations of a body part of a human or an animal. The three-dimensional representation may then be manipulated on a computer using a three-dimensional shape editing program to produce a modified representation of the body part. The modified representation may be used to generate instructions for controlling a carving machine that is configured to directly produce an appliance, or to produce a mold for making appliance, for example. An orthosis is an appliance that is applied externally to a body part to correct deformity, improve function, or relieve symptoms of a disease by supporting on assisting the musculo-neuro-skeletal system. A prosthesis is an appliance that replaces a missing body part. Other appliances such as suppbrting seats or standing shells for supporting the body of a person having limited mobility may also be produced from modified representations of body parts.

The three-dimensional representation of the body part may be produced using a non-contact optical scanner that images the body part with a high level of accuracy. The scanner may include a laser for illuminating the body part with structured light and a video camera for capturing images of the illuminated body part. The captured images may then be processed to extract coordinates of the surface of the body part, which may be used as input coordinates to a computer for producing three-dimensional representations.

It is generally convenient to represent three-dimensional shapes of surfaces using a plurality of two-dimensional slices or planes that permit three-dimensional surface coordinates to be represented and manipulated in two-dimensions. Each surface coordinate location in the two-dimensional plane may be represented using an angle and an offset from a plane origin point. Such representations are usually satisfactory for representing generally cylindrical shapes but may not be satisfactory in representing more complex shapes.

JP2003299679 discloses an artificial limb socket manufacturing system composed of measuring means for measuring a shape of the human body or an object being a model, a design means for designing an artificial limb shape from measuring data, and a laminating molding means for manufacturing the artificial limb by laminating and molding the artificial limb shape.

**SUMMARY OF THE INVENTION**

In accordance with one aspect of the invention there is provided a method for producing a computer representation of a three-dimensional surface of an appliance for a living body. The method involves identifying a plurality of spaced apart planes intersecting the three-dimensional surface and for each plane in the plurality of spaced apart planes identifying a plurality of basis points on the plane. The basis points lie generally along a curve on the plane. The method also involves determining surface coordinate locations of a plurality of points on the plane that lie on the three-dimensional surface, each surface coordinate location being defined as an offset from the basis point, and storing the surface coordinate locations in a computer memory.

The method may involve receiving an input plurality of coordinates defining a preliminary representation of the three-dimensional surface.

The method may involve transforming the surface coordinate locations into a set of instructions operable to control a computer aided manufacturing machine to produce the appliance.

The method may involve applying a shape transformation to the surface coordinate locations to produce modified surface coordinate locations and storing the surface coordinate locations in the computer memory may involve storing the modified surface coordinate locations in the computer memory.

Identifying the plurality of spaced apart planes may involve identifying a plurality of plane coordinate frames respectively identifying locations and orientations of the plurality spaced apart planes in a three-dimensional coordinate system.

Identifying the plurality of plane coordinate frames may involve identifying origin coordinate locations of a plurality of spaced apart points along a reference curve, the reference curve having a shape that generally corresponds to a shape of the three-dimensional surface in a first general direction along the surface, and for each respective origin coordinate location, generating data defining a plane coordinate frame having an origin located at the origin coordinate location and being oriented to cause a corresponding plane defined by the plane coordinate frame to be orthogonally oriented with respect to a portion of the reference curve passing through the origin coordinate location.

The method may involve displaying a representation of the three-dimensional surface, and receiving operator input identifying control point locations defining the reference curve.

Generating the data defining the plane coordinate frame may involve generating data defining a Cartesian coordinate frame having a first axis oriented in a direction along the reference curve at the origin coordinate location and second and third axes defining the orientation of the plane. Identifying the origin coordinate locations may involve identifying regularly spaced apart origin coordinate locations along the reference curve.

Identifying the plurality of plane coordinate frames may involve, for each of the plurality of plane coordinate frames, generating a modeling matrix having elements defining orthogonal unit vectors, the orthogonal unit vectors identifying an orientation of the plane coordinate frame, and elements defining an origin coordinate location of the plane coordinate frame.

Determining the surface coordinate locations may involve determining two-dimensional surface coordinate locations with respect to the plane coordinate frame, and transforming the two-dimensional surface coordinates into three-dimensional coordinates in the three-dimensional coordinate system using the modeling matrix.

The method may involve defining at least one basis curve located on a basis curve plane intersecting the three-dimensional surface, and subdividing the at least one basis curve to identify a plurality of points along the basis curve, and identifying the plurality of basis points on each of the planes may involve projecting respective points in the plurality of points along the basis curve onto each of the planes.

Projecting the respective points may involve at least one of interpolating between points located on at least two basis curves located on respective basis curve planes, and extrapolating from at least one point located on the at least one basis curve.

Defining the at least one basis curve located on the basis curve plane may involve defining at least one basis curve located on one of the spaced apart plurality of planes.

Defining the basis curve may involve defining control points of a B-spline curve located in the plane.

Each the basis curve plane may be defined by a basis curve plane coordinate frame identifying a location and orientation of the plane in a three-dimensional coordinate system and defining the control points of the B-spline curve may involve defining control point coordinate locations in the basis curve plane coordinate frame.

The method may involve displaying a representation of the basis curve plane, and defining the control point coordinate location may involve receiving operator input identifying the control point coordinate locations on the basis curve plane.

Displaying the representation of the basis curve plane may involve displaying an orthographic view of the plane.

Displaying the orthographic view may further involve displaying a polyline representing a shape of the three-dimensional surface in the plane to facilitate selection of the control point locations defining the B-spline curve.

The method may involve determining two-dimensional coordinate locations on the plane of a polyline linking points of intersection between the three-dimensional surface and the plane and determining the offset from each the basis point may involve determining a distance between the basis point and a point of intersection between the polyline and a ray extending from the basis point in a direction normal to the curve.

Determining the two-dimensional coordinate locations on the plane of the polyline may involve determining three-dimensional coordinates of the points of intersection and transforming the points of intersection into two-dimensional coordinates on the plane.

In accordance with another aspect of the invention there is provided an apparatus for producing a computer representation of a three-dimensional surface of an appliance for a living body. The apparatus includes provisions for identifying a plurality of spaced apart planes intersecting the three-dimensional surface, and provisions for identifying a plurality of basis points for each plane in the plurality of spaced apart planes, the basis points lying generally along a curve on the plane. The apparatus also includes provisions for determining surface coordinate locations of a plurality of points on each the plane that lie on the three-dimensional surface, each surface coordinate location being defined as an offset from the basis point, and provisions for storing the surface coordinate locations in a computer memory.

The apparatus may include provisions for receiving an input plurality of coordinates defining a preliminary representation of the three-dimensional surface.

The apparatus may include provisions for transforming the surface coordinate locations into a set of instructions operable to control a computer aided manufacturing machine to produce the appliance.

The apparatus may include provisions for applying a shape transformation to the surface coordinate locations to produce modified surface coordinate locations and the provisions for storing the surface coordinate locations in the computer memory may include provisions for storing the modified surface coordinate locations in the computer memory.

The provisions for identifying the plurality of spaced apart planes may include provisions for identifying a plurality of plane coordinate frames respectively identifying locations and orientations of the plurality spaced apart planes in a three-dimensional coordinate system.

The provisions for identifying the plurality of plane coordinate frames may include provisions for identifying origin coordinate locations of a plurality of spaced apart points along a reference curve, the reference curve having a shape that generally corresponds to a shape of the three-dimensional surface in a first general direction along the surface, and provisions for generating data defining respective plane coordinate frames each having an origin located at respective origin coordinate locations and being oriented to cause a corresponding plane defined by the plane coordinate frame to be orthogonally oriented with respect to a portion of the reference curve passing through the origin coordinate location.

The apparatus may include provisions for displaying a representation of the three-dimensional surface, and provisions for receiving operator input identifying control point locations defining the reference curve.

The provisions for generating the data defining the plane coordinate frame may include provisions for generating data defining a Cartesian coordinate frame having a first axis oriented in a direction along the reference curve at the origin coordinate location and second and third axes defining the orientation of the plane.

The provisions for identifying the origin coordinate locations may include provisions for identifying regularly spaced apart origin coordinate locations along the reference curve.

The provisions for identifying the plurality of plane coordinate frames may include provisions for generating a modeling matrix for each of the plurality of plane coordinate frames, each the modeling matrix having elements defining orthogonal unit vectors, the orthogonal unit vectors identifying an orientation of the plane coordinate frame, and elements defining an origin coordinate location of the plane coordinate frame.

The provisions for determining the surface coordinate locations may include provisions for determining two-dimensional surface coordinate locations with respect to the plane coordinate frame, and provisions for transforming the two-dimensional surface coordinates into three-dimensional coordinates in the three-dimensional coordinate system using the modeling matrix.

The apparatus may include provisions for defining at least one basis curve located on a basis curve plane intersecting the three-dimensional surface, provisions for subdividing the at least one basis curve to identify a plurality of points along the basis curve, and the provisions for identifying the plurality of basis points on each of the planes may include provisions for projecting respective points in the plurality of points along the basis curve onto each of the planes.

The provisions for projecting the respective points may include at least one of provisions for interpolating between points located on at least two basis curves located on respective basis curve planes, and provisions for extrapolating from at least one point located on the at least one basis curve.

The provisions for defining the at least one basis curve located on the basis curve plane may include provisions for defining at least one basis curve located on one of the spaced apart plurality of planes.

The provisions for defining the basis curve may include provisions for defining control points of a B-spline curve located in the plane.

Each the basis curve plane may be defined by a basis curve plane coordinate frame identifying a location and orientation of the plane in a three-dimensional coordinate system and the provisions for defining the control points of the B-spline curve may include provisions for defining control point coordinate locations in the basis curve plane coordinate frame.

The apparatus may include provisions for displaying a representation of the basis curve plane, and the provisions for defining the control point coordinate location may include provisions for receiving operator input identifying the control point coordinate locations on the basis curve plane.

The provisions for displaying the representation of the basis curve plane may include provisions for displaying an orthographic view of the plane.

The provisions for displaying the orthographic view may further include provisions for displaying a polyline representing a shape of the three-dimensional surface in the plane to facilitate selection of the control point locations defining the B-spline curve.

The apparatus may include provisions for determining two-dimensional coordinate locations on the plane of a polyline linking points of intersection between the three-dimensional surface and the plane and the provisions for determining the offset from each the basis point may include provisions for determining a distance between the basis point and a point of intersection between the polyline and a ray extending from the basis point in a direction normal to the curve.

The provisions for determining the two-dimensional coordinate locations on the plane of the polyline may include provisions for determining three-dimensional coordinates of the points of intersection and provisions for transforming the points of intersection into two-dimensional coordinates on the plane.

In accordance with another aspect of the invention there is provided an apparatus for producing a computer representation of a three-dimensional surface of an appliance for a living body. The apparatus includes a processor circuit operably configured to identify a plurality of spaced apart planes intersecting the three-dimensional surface, and for each plane in the plurality of spaced apart planes, to identify a plurality of basis points on the plane, the basis points lying generally along a curve on the plane. The processor circuit is also operably configured to determine surface coordinate locations of a plurality of points on the plane that lie on the three-dimensional surface, each surface coordinate location being defined as an offset from the basis point, and to store the surface coordinate locations in a computer memory.

The processor circuit may be operably configured to receive an input plurality of coordinates defining a preliminary representation of the three-dimensional surface.

The processor circuit may be operably configured to transform the surface coordinate locations into a set of instructions operable to control a computer aided manufacturing machine to produce the appliance.

The processor circuit may be operably configured to apply a shape transformation to the surface coordinate locations to produce modified surface coordinate locations and to store the surface coordinate locations in the computer memory by storing the modified surface coordinate locations in the computer memory.

The processor circuit may be operably configured to identify the plurality of spaced apart planes by identifying a plurality of plane coordinate frames respectively identifying locations and orientations of the plurality spaced apart planes in a three-dimensional coordinate system.

The processor circuit may be operably configured to identify the plurality of plane coordinate frames by identifying origin coordinate locations of a plurality of spaced apart points along a reference curve, the reference curve having a shape that generally corresponds to a shape of the three-dimensional surface in a first general direction along the surface, and for each respective origin coordinate location, generating data defining a plane coordinate frame having an origin located at the origin coordinate location and being oriented to cause a corresponding plane defined by the plane coordinate frame to be orthogonally oriented with respect to a portion of the reference curve passing through the origin coordinate location.

The processor circuit may be operably configured to display a representation of the three-dimensional surface, and receive operator input identifying control point locations defining the reference curve.

The processor circuit may be operably configured to generate the data defining the plane coordinate frame by generating data defining a Cartesian coordinate frame having a first axis oriented in a direction along the reference curve at the origin coordinate location and second and third axes defining the orientation of the plane.

The processor circuit may be operably configured to identify the origin coordinate locations by identifying regularly spaced apart origin coordinate locations along the reference curve.

The processor circuit may be operably configured to identify the plurality of plane coordinate frames by generating a modeling matrix for each of the plurality of plane coordinate frames, the modeling matrix having elements defining orthogonal unit vectors, the orthogonal unit vectors identifying an orientation of the plane coordinate frame, and elements defining an origin coordinate location of the plane coordinate frame.

The processor circuit may be operably configured to determine the surface coordinate locations by determining two-dimensional surface coordinate locations with respect to the plane coordinate frame, and transforming the two-dimensional surface coordinates into three-dimensional coordinates in the three-dimensional coordinate system using the modeling matrix.

The processor circuit may be operably configured to define at least one basis curve located on a basis curve plane intersecting the three-dimensional surface, to subdivide the at least one basis curve to identify a plurality of points along the basis curve, and the processor circuit may be operably configured to identify the plurality of basis points on each of the planes by projecting respective points in the plurality of points along the basis curve onto each of the planes.

The processor circuit may be operably configured to project the respective points by at least one of interpolating between points located on at least two basis curves located on respective basis curve planes, and extrapolating from at least one point located on the at least one basis curve.

The processor circuit may be operably configured to define the at least one basis curve located on the basis curve plane by defining at least one basis curve located on one of the spaced apart plurality of planes.

The processor circuit may be operably configured to define the basis curve by defining control points of a B-spline curve located in the plane.

Each basis curve plane may be defined by a basis curve plane coordinate frame identifying a location and orientation of the plane in a three-dimensional coordinate system and the processor circuit may be operably configured to define the control points of the B-spline curve by defining control point coordinate locations in the basis curve plane coordinate frame.

The processor circuit may be operably configured to display a representation of the basis curve plane and to define the control point coordinate location by receiving operator input identifying the control point coordinate locations on the basis curve plane.

The processor circuit may be operably configured to display the representation of the basis curve plane by displaying an orthographic view of the plane.

The processor circuit may be operably configured to display the orthographic view by displaying a polyline representing a shape of the three-dimensional surface in the plane to facilitate selection of the control point locations defining the B-spline curve.

The processor circuit may be operably configured to determine two-dimensional coordinate locations on the plane of a polyline linking points of intersection between the three-dimensional surface and the plane and to determine the offset from each the basis point by determining a distance between the basis point and a point of intersection between the polyline and a ray extending from the basis point in a direction normal to the curve.

The processor circuit may be operably configured to determine the two-dimensional coordinate locations on the plane of the polyline by determining three-dimensional coordinates of the points of intersection and by transforming the points of intersection into two-dimensional coordinates on the plane.

In accordance with another aspect of the invention there is provided a computer readable medium encoded with codes for directing a processor circuit to produce a computer representation of a three-dimensional surface of an appliance for a living body. The codes direct the processor circuit to identify a plurality of spaced apart planes intersecting the three-dimensional surface, and for each plane in the plurality of spaced apart planes to identify a plurality of basis points on the plane, the basis points lying generally along a curve on the plane. The codes also direct the processor circuit to determine surface coordinate locations of a plurality of points on the plane that lie on the three-dimensional surface, each surface coordinate location being defined as an offset from the basis point, and to store the surface coordinate locations in a computer memory.

In accordance with another aspect of the invention there is provided a computer readable signal encoded with codes for directing a processor circuit to produce a computer representation of a three-dimensional surface of an appliance for a living body. The codes direct the processor circuit to identify a plurality of spaced apart planes intersecting the three-dimensional surface, and for each plane in the plurality of spaced apart planes to identify a plurality of basis points on the plane, the basis points lying generally along a curve on the plane. The codes also direct the processor circuit to determine surface coordinate locations of a plurality of points on the plane that lie on the three-dimensional surface, each surface coordinate location being defined as an offset from the basis point, and to store the surface coordinate locations in a computer memory.

Other aspects and features of the present invention will become apparent to those ordinarily skilled in the art upon review of the following description of specific embodiments of the invention in conjunction with the accompanying figures.

## BRIEF DESCRIPTION OF THE DRAWINGS

In drawings which illustrate embodiments of the invention,

Figure **1**      is a perspective view of a system for producing a computer representation of a three-dimensional surface of an appliance for a living body in accordance with a first embodiment of the invention;

Figure **2**      is a block diagram of a processor circuit used in the system shown in Figure **1**;

Figure **3**      is a flowchart of blocks of codes for directing the processor circuit shown in Figure **2** to execute a process for producing a computer representation;

Figure **4**      is a perspective representation of a three-dimensional surface of an appliance for a living body;

Figure **5**      is a polygon mesh representation of the three-dimensional surface of the appliance shown in Figure **4**;

Figure **6**      is an orthogonal view of a plane shown Figure **4**;

Figure **7**      is a flowchart of blocks of codes for directing the processor circuit shown in Figure **2** to execute a process for identifying a plurality of planes shown in Figure **4**;

Figure **8**      is a screenshot of an operator interface produced for display by the processor circuit shown in Figure **2**;

Figure **9**      is a perspective view of a portion of a reference curve shown in Figure **8**;

Figure **10**      is a flowchart of blocks of codes for directing the processor circuit shown in Figure **2** to execute a process for defining basis points;

Figures **11 - 13**      are a series of screenshots of views produced for display by the processor circuit shown in Figure **2**; and

Figure **14**      is further orthogonal view of the plane shown Figure **4**;

Figure **15** is a flowchart of blocks of codes for directing the processor circuit shown in Figure **2** to execute a process for determining surface coordinate locations.

**DETAILED DESCRIPTION**

System overview

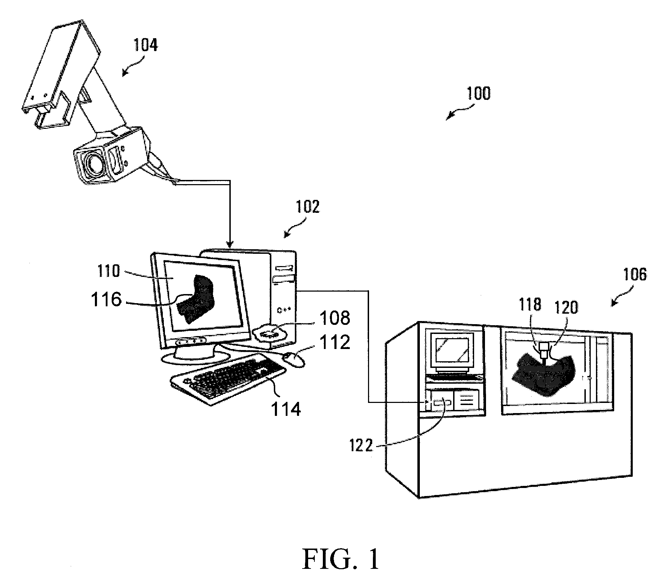

Referring to Figure **1,** a CAD/CAM (computer aided design/computer aided manufacturing) system for producing a computer representation of a three-dimensional surface of an appliance for a living body is shown generally at **100.** The system **100** includes a CAD apparatus **102,** a scanner **104,** and a computer aided manufacturing (CAM) machine **106.**

The apparatus **102** is in communication with the scanner **104** for receiving a signal encoded with an input plurality of coordinates representing a general shape of a part of a living body for which the appliance is to be produced. The scanned body part may be any body part, or group of body parts in any particular orientation for which it is desired to produce an appliance. For example, the scanned body part may be posterior portion of a human patient's torso and legs and the scanned input coordinates may be used to produce a supporting seat appliance for supporting the patient's body in a seated position.

Examples of suitable scanners include the FastSCAN Cobra handheld scanner manufactured by Polhemus of Colchester, Vermont, the Yeti Foot Scanner manufactured by Vorum Research Corporation of British Columbia, Canada, and the STARscanner™ manufactured by Orthomerica Products Inc. of California.

The apparatus **102** includes a processor circuit **108** for receiving the input plurality of coordinates and for producing the computer representation of the three-dimensional surface of the intended appliance. The input plurality of coordinates may be produced from coordinate points received from the scanner **104,** for example. The apparatus **102** also includes a display **110** in communication with the processor circuit **108.** In this embodiment the apparatus **102** also includes a pointing device **112** having one or more actuator buttons for receiving operator input from an operator of the apparatus and a keyboard **114** for receiving alphanumeric input from the operator. The processor circuit **108** produces signals for causing the display **110** to display representations of a surface of the appliance being produced and to provide interactive visual feedback during modification of the appliance by an operator in response to inputs received at the pointing device **112** and keyboard **114** for producing a final appliance. In this case, a representation of a support appliance **116** for receiving a posterior portion of a patient's body is displayed on the display **110**.

In alternative embodiments the input plurality of coordinates may be provided from a library of standard shapes of previously scanned appliances which are may be scaled and otherwise modified to provide a final appliance that fits a particular patient.

In general, producing an appliance for a patient involves receiving the input plurality of coordinates defining a preliminary representation of the surface of the appliance and transforming the input plurality of coordinates into a computer representation that facilitates modifications to the shape of the appliance. Accordingly, the final appliance representation may include alterations to the shape of surfaces, such as compressions in areas of the body that tolerate pressure and/or relief in certain areas of the body that are sensitive to pressure, thus providing a comfortably fitting appliance as defined by the final appliance representation.

The CAM machine **106** generally includes a machine tool portion **118** for machining the appliance. In this case the machined appliance is a mold **120** which is subsequently used to produce a final appliance by molding a thermoplastic or other material over the mold. The machined mold **120** has a shape defined by the computer representation of the mold and generally corresponds to the shape of the scanned body part, with alterations for fit, comfort, and/or support.

The CAM machine **106** also includes a controller **122** for controlling the machine tool portion **118** of the CAM machine. The controller **122** is in communication with the apparatus **102** for receiving a signal encoded with instructions operable to control the CAM machine **106** to produce the machined appliance **120.** Alternatively the instructions may be output to a computer readable medium or memory for manual transfer to the CAM machine **106.** An example of suitable CAM machines are the CANFIT-PLUS™ Carver and the CANFIT™ **6**-Axis Carver, both produced by Vorum Research Corporation of British Columbia, Canada.

Processor Circuit

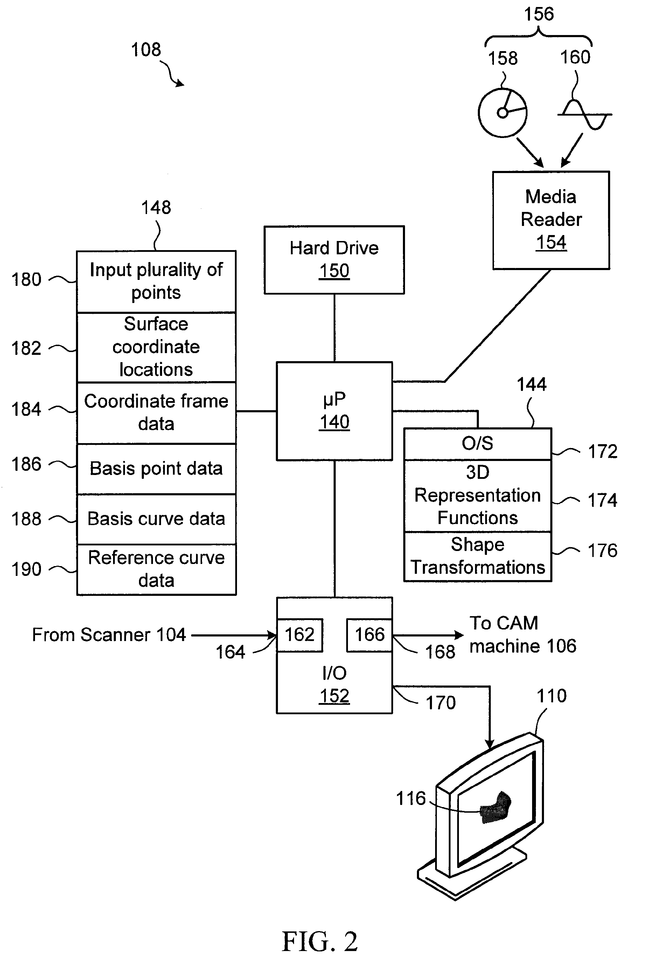

The processor circuit **108** of the apparatus **102** is shown in greater detail in Figure **2.** Referring to Figure **2,** the processor circuit **108** includes a microprocessor **140,** a program memory **144,** a random access memory (RAM) **148,** a hard drive **150,** an input output port (I/O) **152,** and a media reader **154,** all of which are in communication with the microprocessor **140.**

Program codes for directing the microprocessor **140** to carry out various functions are stored in the program memory **144,** which may be implemented as a random access memory (RAM), and/or a hard disk drive (HDD), or a combination thereof. The program memory **144** includes a block of codes **172** for directing the microprocessor to provide general operating system (O/S) functions, and a block of codes **174** for directing the microprocessor **140** to provide functions for producing the computer representation of the three-dimensional surface of the appliance. In this embodiment, the program memory **144** also includes a block of codes **176** for directing the microprocessor **140** to provide shape representation functions for modifying the computer representation of the appliance to produce the final appliance.

The media reader **154** facilitates loading program codes into the program memory **144** from a computer readable medium **156,** such as a CD ROM disk **158,** a flash memory (not shown), or a computer readable signal **160** such as may be received over a network such as the internet, for example. The media reader also facilitates receiving the input plurality of coordinates and/or outputting carving instructions to the computer readable medium **156** for manual transfer to the CAM machine **106.**

The RAM **148** includes a plurality of storage locations including a store **180** for storing the input plurality of coordinates, a store **182** for storing surface coordinate locations, a store **184** for storing a coordinate frame data, a store **186** for storing basis point data, a store **188** for storing basis curve data, and a store **190** for storing reference curve data.

The hard drive **150** includes a plurality of storage locations for persistent storage of data, such as library shapes and operating system files, for example.

The I/O **152** includes a first interface **162** having an input **164** for receiving the signal encoded with the input plurality of coordinates from the scanner **104,** and a second interface **166** having an output **168** for producing the signal encoded with the instructions for controlling the CAM machine **106** to produce the appliance. The interfaces **162** and **166** may be universal serial bus (USB) or an RS**232** serial interface for example. The I/O **152** also includes an output **170** for producing a display signal for causing a representation of the appliance **116** to be displayed on the display **110.**

Operation

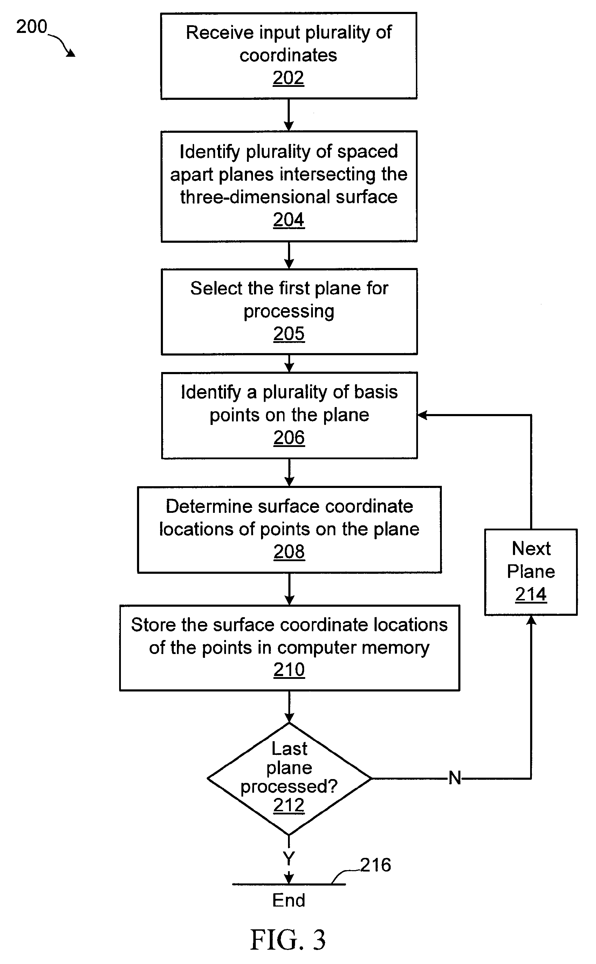

The operation apparatus **102** shown in Figure **1** and Figure **2** to produce the computer representation of the three-dimensional surface of the appliance is described with reference to Figures **3** to **7.**

Referring to Figure **3,** a flowchart depicting blocks of code for directing the microprocessor **140** (shown in Figure **2**) to execute a process for producing the computer representation is shown generally at **200.** The process begins at block **202**, which directs the microprocessor **140** to receive an input plurality of coordinates defining a preliminary representation of the three-dimensional surface.

In one embodiment the input plurality of coordinates may be provided by scanning the patient's body using the scanner **104** shown in Figure **1.** In other embodiments the input plurality of coordinates may be read from a library of appliance shapes stored on the hard drive **150.** Block **202** also directs the microprocessor **140** to store the input plurality of coordinates in the store **180** of the RAM **148** (shown in Figure **2**).

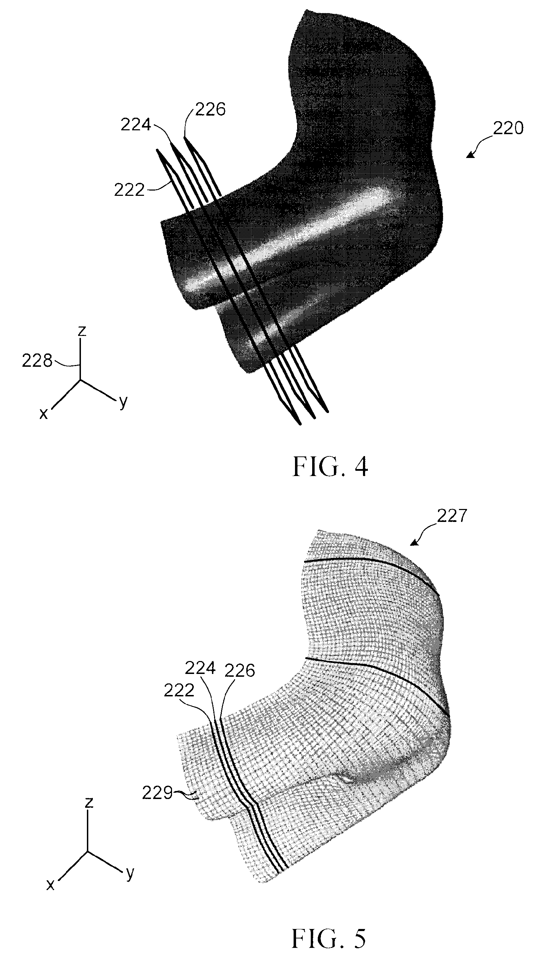

Referring to Figure **4,** a preliminary representation of a shaded three-dimensional surface of a support appliance is shown generally at **220.** In this embodiment the input plurality of coordinates are defined using coordinates in a Cartesian coordinate system as indicated by coordinate axes x, y, and z shown at **228.**

Referring back to Figure **3,** block **204** then directs the microprocessor **140** to identify a plurality of spaced apart planes intersecting the preliminary shape representation **220.** Referring again to Figure **4,** three exemplary planes are shown including a first plane **222,** a second plane **224,** and a third plane **226.** In practice a sufficiently large plurality of spaced apart planes would be identified to represent the preliminary shape in sufficient detail. In general, identifying the plurality of spaced apart planes **222 - 226** involves defining coordinates of the planes in a coordinate space such as the Cartesian coordinate system **228,** such as for example defining plane coordinate frames defined within the Cartesian coordinate space **228,** as described later herein.

In some embodiments, the input plurality of coordinates may be received in a format that already has defined planes (such as the planes **222 - 226**). For example, referring to Figure **5,** the shape representation **220** may be received as a surface polygon mesh **227,** having vertices lying in spaced apart planes defining a plurality of rectangles **229.** In this case, the planes **222, 224,** and **226** (shown bolded) may be used as provided by the surface mesh. However, more commonly the preliminary shape representation will require processing to produce the planar mesh shape shown in Figure **5**, as described later herein. For example, a common file format output by many scanners (such as the scanner **104**) is StL ("StereoLithography") file format, which uses triangular polygons to represent three-dimensional surfaces. Accordingly, the triangular polygons would have to be re-sampled to produce the planar mesh shown in Figure **5.**

Referring back to Figure **3,** the process **200** then continues at block **205,** which directs the microprocessor **140** to select the first identified plane for processing.

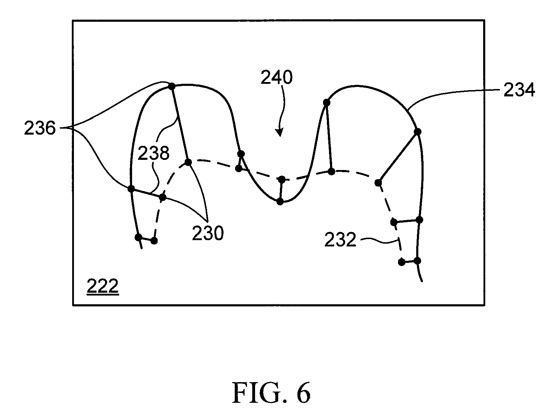

Block **206** then directs the microprocessor **140** to identify a plurality of basis points on the first plane **222.** Referring to Figure **6,** the plane **222** is shown in orthogonal (plan) view, and includes a plurality of basis points **230** lying generally along a curve **232.** A polyline **234** is shown linking points of intersection between the shape representation **220** and the plane **222.** The polyline **234** is made up of a plurality of line segments and the curve **232** generally has a shape that at least generally follows some portions of the polyline **234.** In the embodiment shown, the curve **232** is not closed since the outline of the shape being represented is a shell shape. However, in other embodiments the curve **232** may have closed ends for representing a closed shape, such as a prosthesis, for example.

Referring back to Figure **3,** block **208** then directs the microprocessor **140** to determine surface coordinate locations of a plurality of points on the plane that lie on the preliminary shape representation **220**. Referring again to Figure **6,** a plurality of points **236** lying on the polyline outline **234** have surface coordinate locations defined by respective offsets **238** from the respective basis points **230**. In the embodiment shown, the offsets **238** are taken in a direction normal to the curve **232**. Determining locations of the points **236** on the plane **222** may involve interpolating between vertices of the surface representation lying in proximity to the plane **222,** for example. Referring back to Figure **3,** the process then continues at block **210** which directs the microprocessor **140** to store the surface coordinate locations in the store **182** of the RAM **148.**

The process then continues at block **212,** which directs the microprocessor **140** to determine whether the last plane has been processed. If there are still planes left in the plurality of planes that have not yet been processed then the process continues at block **214,** which directs the microprocessor **140** to select the next plane for processing. Block **214** also directs the microprocessor **140** back to block **206** and blocks **206 - 212** are repeated for each successive plane (such as the planes **224** and **226** shown in Figure **4**).

If at block **212,** there are no more planes remain that are still to be processed, then the process **200** ends at **216.**

Advantageously, by representing surface coordinate locations of points on the shape representation **220** (shown in Figure **4**) using offsets from a plurality of basis points provides for an accurate shape representation. Referring back to Figure **6,** prior art shape representations may have difficulty in accurately representing regions such as the region shown generally at **240,** and in such cases surface coordinates may end up being sparsely represented in these regions. Advantageously, using the basis points **230** along the curve **232** facilitates identification of a sufficiently dense number of surface coordinate locations in the region **240** to permit accurate representation of even complex shapes.

Furthermore, representation of surface coordinate locations using spaced apart planes facilitates simple modification of the appliance, for example by scaling along a plane to change the size of the appliance, or by changing the spacing and/or relative orientation between planes to alter the shape of the appliance. Such shape modifications may be advantageously performed by an operator who is not necessarily expert in three-dimensional CAD, as the spaced apart planes facilitate intuitive shape transformations without requiring in-depth knowledge of three-dimensional geometric transformations.

Identifying planes

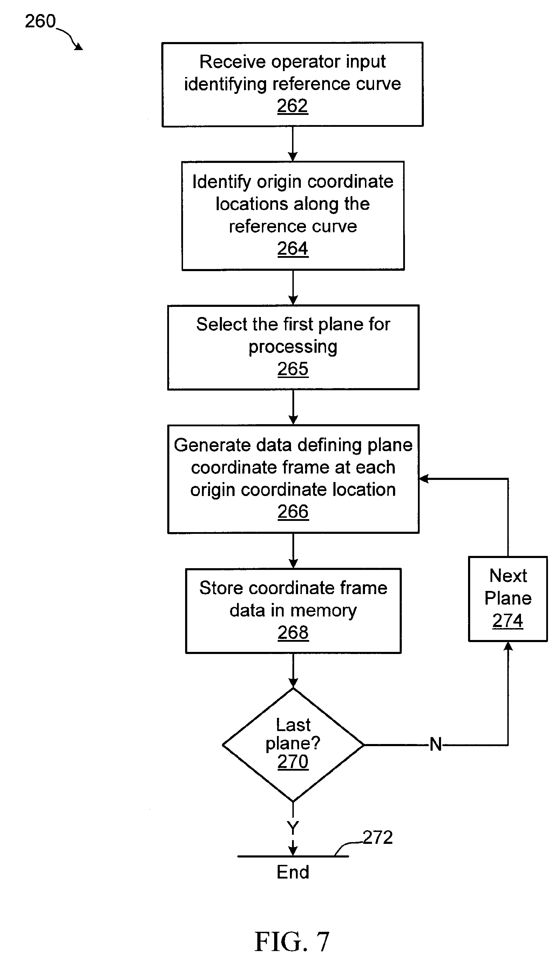

One operational embodiment of block **204** of the process **200** is shown in greater detail in Figure **7.** Referring to Figure **7,** a flowchart depicting blocks of code for directing the microprocessor **140** to execute a process for identifying the plurality of spaced apart planes is shown generally at **260.**

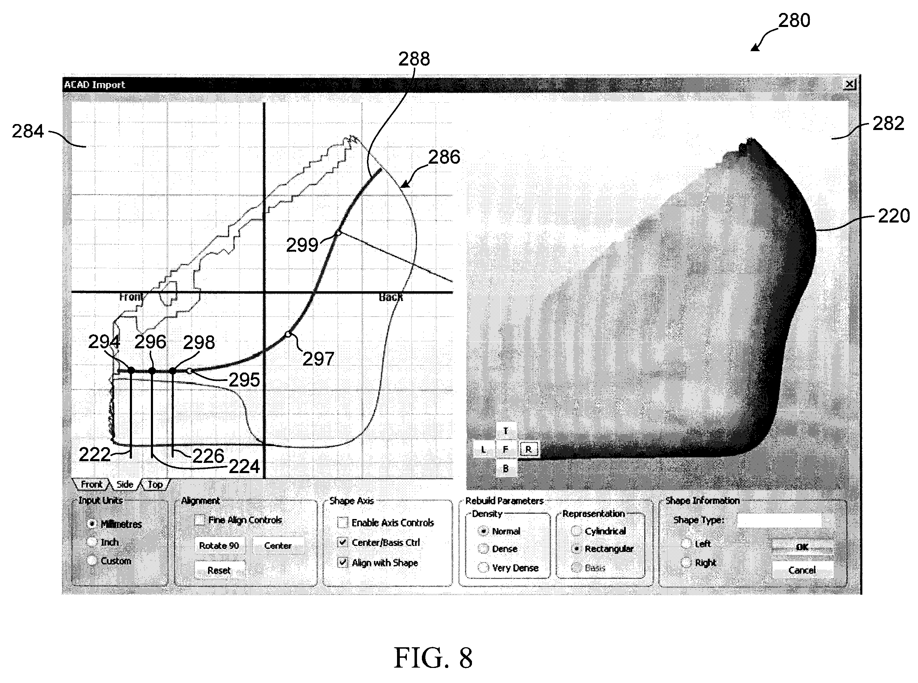

The process **260** begins at block **262,** which directs the microprocessor **140** to display a screen for receiving operator input identifying a reference curve. Referring to Figure **8,** a screenshot showing a side view of the shape representation **220** is shown generally at **280.** The screenshot **280** represents an operator interface for receiving input from an operator for identifying the plurality of spaced apart planes **222 - 226** as shown in Figure **4.** The screenshot **280** includes a window **282** for viewing a shaded version of the preliminary shape representation **220,** and a window **284** for viewing an outline shape **286** of the shaded shape representation **220** shown in the window **282.** In general, shading the three-dimensional shape representation involves shading underlying polygons making up the shape using a shading algorithm such as Gouraud shading.

The window **284** also displays a reference curve **288.** The reference curve **288** may be identified in response to operator input received at the pointing device **112** and/or keyboard **114** (shown in Figure **1**) to identify control points (not shown) of a reference curve, such as a B-spline curve. The control points may then be interactively positioned and adjusted by the operator to achieve a desired shape of the reference curve **288.** The operator may also move control points to cause the reference curve **288** to be shaped to generally correspond to one or more features of the three-dimensional surface that it is desired to accurately represent. For example, the reference curve **288** may generally follow an anatomical axis of the body part or parts that the appliance is to support.

Referring back to Figure **7,** block **262** also directs the microprocessor **140** to store data defining the reference curve (such as control point and/or endpoint coordinate locations) in the reference curve data store **190** of the RAM **148** shown in Figure **2.**

Block **264** then directs the microprocessor **140** to identify origin coordinate locations along the reference curve **288.** In the embodiment shown, the reference curve **288** is subdivided to produce a plurality of regularly spaced apart origin coordinate locations, such as the three coordinate locations **294, 296,** and **298** shown. While only three origin coordinate locations are shown, it should be understood that further origin coordinate locations will generally be identified along the reference curve **288.** In this embodiment the reference curve is subdivided to produce regularly spaced apart origin coordinate locations **294, 296,** and **298** having sufficient density to permit accurate representation of the three-dimensional surface.

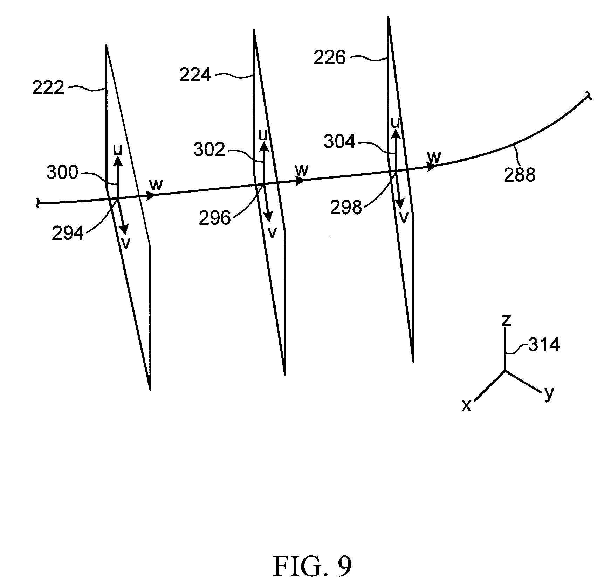

The process 260 then continues at block 265, which directs the mircroprocessor 140 to select the first plane for processing. Block **266** then directs the microprocessor **140** to generate data defining a plane coordinate frame at each origin location. In general, the plane coordinate frames are defined in a three-dimensional coordinate system (for example the Cartesian coordinate system shown in Figure **4)** and generally define locations of the spaced apart plurality of planes **222 - 226.** Referring to Figure **9,** a portion of the reference curve **288** is shown in greater detail. Each of the origin coordinate locations **294, 296,** and **298,** are shown having a respective coordinate frame **300, 302,** and **304.** The coordinate frames **300, 302,** and **304** are each oriented orthogonally with respect to the reference curve **288** at the origin coordinate locations **294, 296,** and **298.** The w-axis of each coordinate frame **300, 302,** and **304** is oriented along the reference curve and the uv-axes define the respective planes **222, 224,** and **226.**

The coordinate frames **300, 302,** and **304** are in turn defined in the (x,y,z) three-dimensional coordinate system shown at **314.** In the embodiment shown the coordinate frames **300, 302,** and **304** are defined as Cartesian coordinate frames, but in other embodiments alternative coordinate systems may be used to define the coordinate frames. Advantageously, all points lying on the u-v axes (i.e. the planes **222 - 226)** are two-dimensional coordinates and have a w coordinate value of zero.

In one embodiment, the coordinate frames **300, 302,** and **304** are defined by a three-dimensional modeling matrix:

$$M = \begin{bmatrix} a_{11} & a_{12} & a_{13} & 0 \\ a_{21} & a_{22} & a_{23} & 0 \\ a_{31} & a_{32} & a_{33} & 0 \\ a_{41} & a_{42} & a_{43} & 1 \end{bmatrix}, \qquad \text{Eqn } \mathbf{1}$$

where the elements $a_{11}$, $a_{12}$, and $a_{13}$ represent a unit vector defining the u-axis of the coordinate frame, $\mathbf{a}_{21}$, $a_{22}$, and $a_{23}$ represent a unit vector defining the v-axis of the coordinate frame, and $a_{31}$, $a_{32}$, and $a_{33}$ represent a unit vector defining the w-axis of the coordinate frame. The elements $a_{41}$, $a_{42}$, and $a_{43}$ represent x, y, and z values of the origin coordinate location for in the (x,y,z) coordinate system **314** of each of the respective coordinate frames **300, 302,** and **304.** The modeling matrix $M$ may be used to transform two-dimensional surface coordinates lying on the u-v plane into three-dimensional coordinates defined in the three-dimensional coordinate system **314.** Each two-dimensional u-v plane coordinate may be represented by the vector:

$$\overline{P} = \begin{bmatrix} u & v & 0 & 1 \end{bmatrix} \qquad \text{Eqn } \mathbf{2}$$

where the w coordinate is zero everywhere on the planes **222 - 226.** Similarly, three-dimensional coordinates may be represented by the vector:

$$\overline{Q} = \begin{bmatrix} x & y & z & 1 \end{bmatrix} \qquad \text{Eqn } \mathbf{3}$$

Transforming two-dimensional plane coordinates into three-dimensional (x,y,z) coordinates thus involves, the following matrix multiplication:

$$\overline{Q} = \overline{P}M . \qquad \text{Eqn } \mathbf{4}$$

Conversely, transforming three-dimensional (x,y,z) surface coordinates into two-dimensional plane coordinates involves the following matrix multiplication:

$$\overline{P} = \overline{Q}M^{-1}.$$

Eqn 5

Advantageously, the above coordinate transformations permit the appliance to be conveniently modified in the two-dimensional *uv*-axis planes or by relocating the coordinate frames **300, 302,** and **304** with respect to each other and then transformed into three-dimensional (x,y,z) coordinates for producing the final appliance using the CAM machine **106** shown in Figure **1**.

Referring back to Figure **7,** the process **260** then continues at block **268,** which directs the microprocessor **140** to store the data defining the plane coordinate frame (i.e. elements of the modeling matrix M) in the store **184** of the RAM **148** (shown in Figure **2**). Block **270** then directs the microprocessor **140** to determine whether the last coordinate frame for the last of the plurality of origin coordinate locations along the reference curve **288** has been identified, in which case the process ends at **272**.

If at block **270,** the last coordinate frame for the last of the plurality of origin coordinate locations along the reference curve **288** has not yet been identified, then the microprocessor **140** is directed to block **274**. Block **274** directs the microprocessor **140** to select the next plane for processing and directs the microprocessor **140** to return to block **266** and repeat blocks **266 - 270.**

Defining basis points

Referring back to Figure **8,** in one embodiment identifying the basis points (i.e. block **206** shown in Figure **3**) generally involves defining one or more basis curves along the reference curve **288** and projecting the basis curve onto the planes **222- 226**. In the embodiment shown, three basis curve plane locations **295, 297,** and **299** are shown.

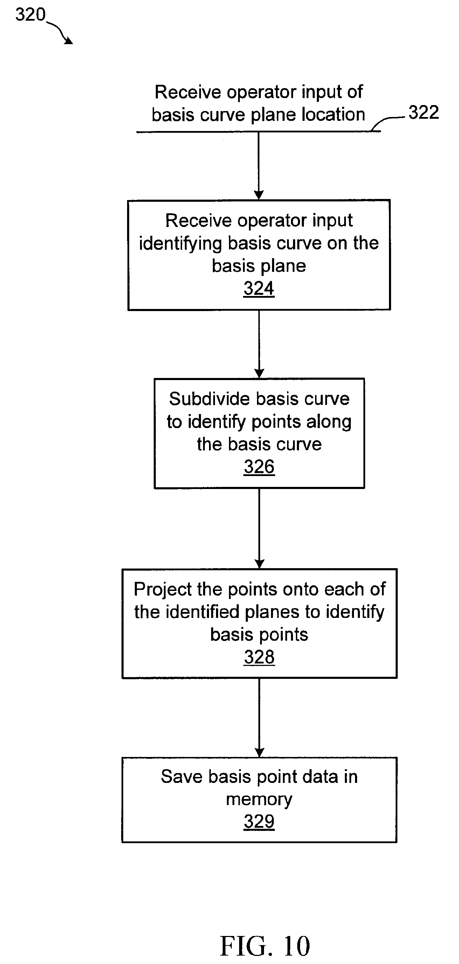

Referring to Figure **10,** a flowchart depicting blocks of code for directing the processor circuit **140** to execute a process for indentifying the basis points in the planes **222 - 226** is shown generally at **320**. In one embodiment, the process **320** may be executed after defining the reference curve **288,** as described above and before identifying the plurality of spaced apart planes.

The process begins at block **322,** when operator input is received indicating a new basis curve plane location along the reference curve **288**. Referring back to Figure **8,** in the embodiment shown, three basis curve plane locations **295, 297,** and **299** are identified. The basis curve planes are not necessarily coplanar with any of the spaced apart plurality of planes (for example the planes **222 - 226**) and may be located anywhere along the reference curve **288**. However, in this embodiment the basis curve planes are also defined by a coordinate frame (not shown) in the same way as described above in connection with the spaced apart planes **222 - 226**.

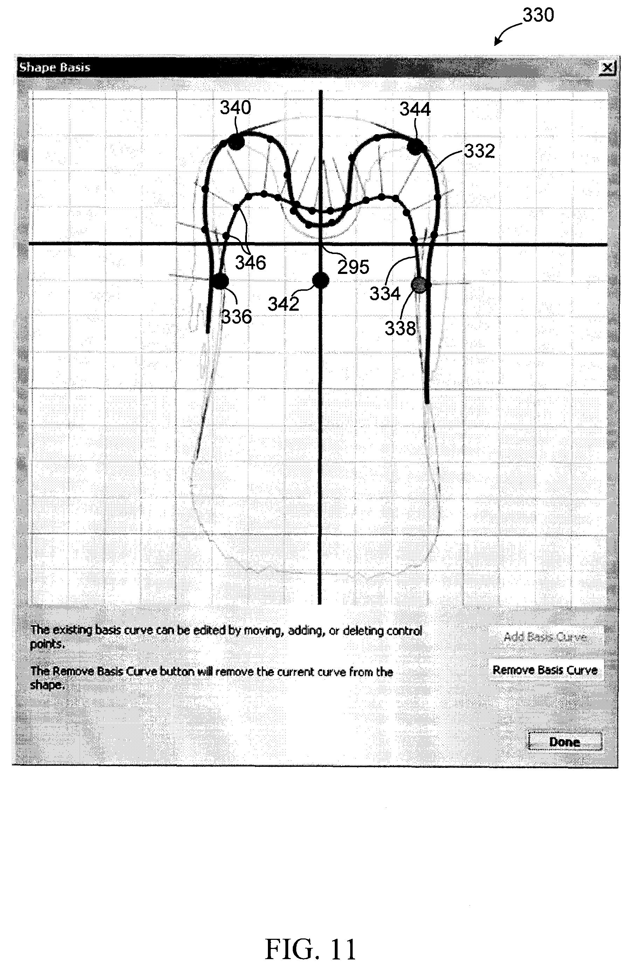

Referring to Figure **11**, a screenshot of a user interface window displayed in response to the operator clicking on the basis curve plane location **295** is shown generally at **330**. The window **330** generally provides an orthographic view of a basis curve plane at the location **295**. The window **330** includes a displayed polyline outline **332**, which indicates points of intersection between the polygons of the three-dimensional preliminary shape and the basis curve plane.

The process **320** then continues at block **324,** which directs the microprocessor **140** to receive operator input identifying a basis curve **334** on the basis curve plane displayed in the window **330**. In this embodiment, the basis curve **334** comprises a B-spline curve having endpoints **336** and **338,** and having a shape defined by control points **340, 342,** and **344.** When the window **330** is first displayed for the operator, the basis curve **334** may be defined as an initial quadratic or cubic B-spline curve located in a default position with respect to the basis curve plane origin coordinate location **295**. The operator may then drag the endpoints **336** and **338** and control points **340, 342,** and **344** to define a desired shape and location of the basis curve. The operator may also click on the displayed screen to add further control points to the B-spline curve. In general B-spline curves may be represented by two or more control points (i.e. endpoints **336** and **338** and zero or more additional control points). B-spline curves with three control points are usually quadratic Bezier curves, and with four control points are usually cubic Bezier curves. Higher order shapes having more than four control points are generally represented as smoothly connected Bezier curves of lower order.

Referring back to Figure **10**, block **324** also directs the microprocessor **140** to store coordinates of the locations of the endpoints **336** and **338** and control points **340, 342,** and **344** in the basis curve store **188** of the RAM **148** (shown in Figure **2**). As in the case of the spaced apart planes **222 - 226,** the saved coordinate locations may be defined as two-dimensional coordinates referenced to a basis curve plane coordinate frame.

The process then continues at block **326,** which directs the microprocessor **140** to subdivide the basis curve **334** to identify a plurality of points **346** (shown in Figure **11**) along the basis curve. The number of points **346** is generally selected to provide a sufficiently dense representation of the three-dimensional surface, and in one embodiment the number of points identified may be about **60** points.

Block **328** then directs the microprocessor **140** to project the points identified at block **326** onto the spaced apart planes **222** - **226** to determine locations of the basis points on each plane (i.e. the basis points **230** shown in Figure **6**). In embodiments where only one basis curve plane location **295** is identified, the basis points in each of the spaced apart planes **222** - **226** will lie along a curve having the same shape and location as the basis curve shown at **334** in Figure **11**.

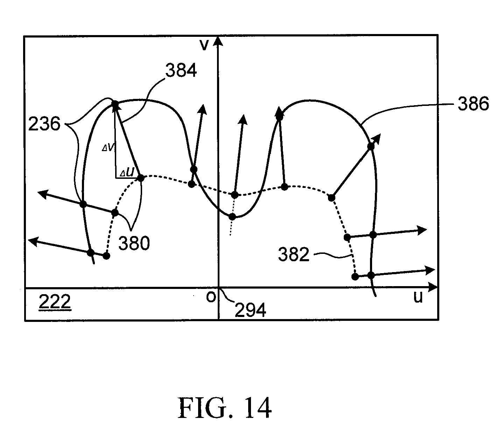

Referring to Figure **14**, the plane **222** is again shown in orthogonal (plan) view, including a plurality of basis points **380** along a curve **382**. Each basis point **380** has a ray **384** located at the basis point. In this embodiment the ray **384** is normal to the curve **382** at the respective basis point. The basis point **380** may thus be represented as a unit vector or ray having elements defined as follows:

$$\overline{R} = \begin{bmatrix} u & v & \Delta u & \Delta v \end{bmatrix} \qquad \text{Eqn } 6$$

where *u* and *v* are the coordinates of the basis point **230** in the *uv*-axes and $\Delta u$ and $\Delta v$ provide a slope or gradient defining the direction of the ray.

Referring back to Figure **10**, the process **320** then continues at block **329**, which directs the microprocessor **140** to store element values of the ray $\overline{R}$ for each of the identified basis points **380** in the store **186** of the RAM **148** (shown in Figure **2**).

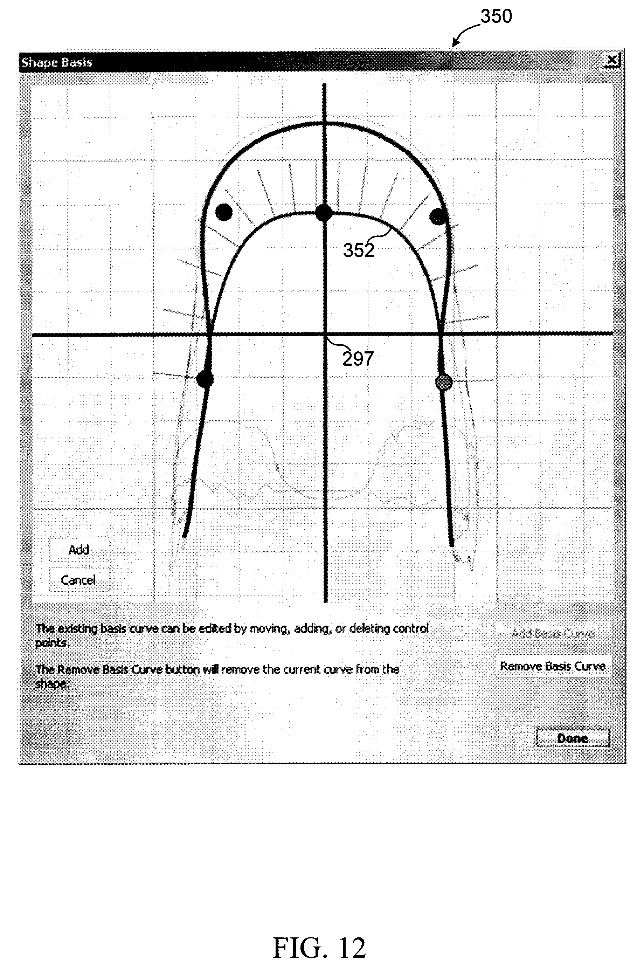

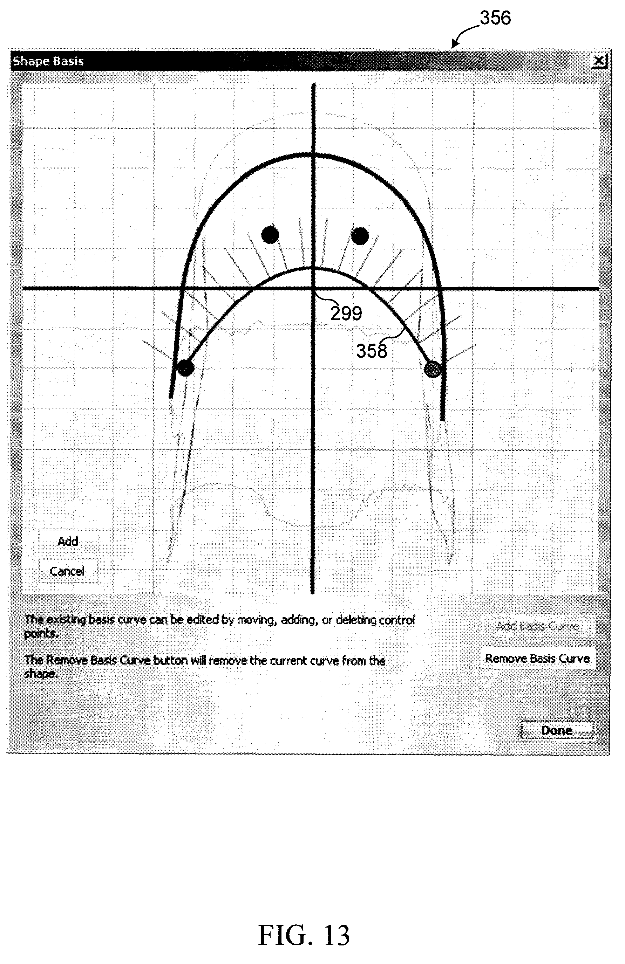

In the embodiment shown in Figure **8**, three basis curve plane locations **295**, **297**, and **299** are shown. The process **320** may then be executed for each of the remaining basis curve plane locations **297** and **299** as described above for the basis curve plane location **295**. Referring to Figure **12** and Figure **13**, screenshots of respective user interface windows for receiving user input defining respective basis curves **352** and **358** are shown at **350** and **356** respectively.

Referring back to Figure **10**, each time a new basis curve is added (for example at the basis curve plane location **297**), block **328** directs the microprocessor **140** to project the points onto the spaced apart planes **222** - **226**. As described above, when only a single basis curve plane location is defined, point locations are duplicated across the plurality of spaced apart planes to produce corresponding basis points on each of the planes. When two or more basis curve plane locations are identified, locations of basis points on the spaced apart planes are interpolated between corresponding points on each of the two defined basis curves for planes in-between the defined basis curves and extrapolated for other planes not located in-between. In this embodiment the interpolation/extrapolation is performed using a cubic function or other non-linear interpolation methods. In general the set of basis curves, when projected onto the spaced apart planes may be viewed as together defining a basis surface, which forms a template shape for producing the representation of the three-dimensional surface.

In other embodiments, basis curve plane locations **295, 297,** and **299** may be constrained to lie on one of the spaced apart plurality of planes such as the planes **222 - 226** shown in Figure **8.**

Determining surface coordinate locations

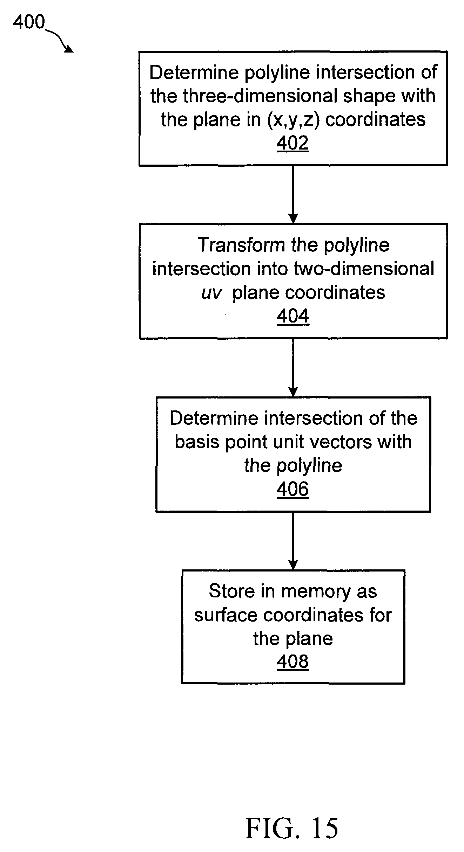

One embodiment of the block **208** of the process **200** shown in Figure **3** for determining surface coordinate locations is shown in greater detail at **400** in Figure **15**.

The process **400** begins at block **402**, which directs the microprocessor **140** to determine the intersection of the preliminary three-dimensional shape with the plane (i.e. one of the spaced apart planes such as the plane **222**). As stated above, the preliminary three-dimensional surface of the appliance is generally represented using a collection of flat polygons, a subset of which will each intersect the plane **222** at two edges of the polygon thus defining pairs of points making up line segments. Coordinates of the points the three-dimensional (x,y,z) coordinate system may then be interpolated from the coordinate locations of the vertices of the flat polygons to yield a set of (x,y,z) points defining a polyline of intersection between the three-dimensional shape and the plane **222**.

Block **404** then directs the microprocessor **140** to transform the polyline coordinates from three-dimensional (x,y,z) coordinates into two-dimensional coordinates on the plane **222** using equation **5** above. The transformation results in a plurality of points **380** having coordinates defined in the plane **222**.

The process **400** then continues at block **406**, which directs the microprocessor **140** to determine points of intersection of the basis point rays **384** (read from the store **186** of the RAM **148** in Figure **2**) on the plane **222**. Referring back to Figure **14**, a general shape of the polyline of the intersection with the plane is shown at **386**. By determining intersections between the rays **384** and the polyline **386**, a plurality of surface coordinate locations **188** may be determined on the plane. The intersection coordinate locations will not necessarily lie at the two-dimensional polyline points transformed in block **404**, and interpolation will be required between points on the polyline to determine the surface coordinate locations along the polyline.

Block **408** then directs the microprocessor **140** to store the surface coordinate locations <u>**188**</u> in the store **182** of the RAM **148.** Since the surface coordinate locations are determined with reference to the basis points **380,** in one embodiment surface coordinate locations may be stored as an offset distance and a reference to the corresponding basis point **380.** Accordingly, to identify each surface coordinate locations on the plane would require only a reference to the basis point and an offset therefrom to be stored in the store **182.** Basis point coordinate locations are stored in the store **186** and may be conveniently referenced to avoid duplication.

Advantageously, by using basis curves and/or basis points to provide a template for representing a three-dimensional shape of the appliance facilitates representation of complex shapes by permitting the basis curve shape to be selected as a template. The template shape may be selected to permit accurate representation of all portions of the appliance.

While specific embodiments of the invention have been described and illustrated, such embodiments should be considered illustrative of the invention only and not as limiting the invention as construed in accordance with the accompanying claims.

**Claims**

1. A method for producing a computer representation of a three-dimensional surface (220,227) of an appliance (120) for a living body, the method comprising identifying a plurality of spaced apart planes (222,224,226) intersecting the three-dimensional surface, the method **characterized by**:

   for each plane in said plurality of spaced apart planes:

   identifying a plurality of basis points (230,346,380) on said plane, said basis points lying generally along a curve (232,334,352,358,382) on said plane;
   determining surface coordinate locations (236) of a plurality of points on said plane that lie on said three-dimensional surface, each surface coordinate location being defined as an offset (238,384) from one of said plurality of basis points; and
   storing said surface coordinate locations in a computer memory (148).

2. The method of claim **1** further comprising transforming said surface coordinate locations into a set of instructions operable to control a computer aided manufacturing machine (106) to produce the appliance.

3. The method of claim **1** further comprising applying a shape transformation to said surface coordinate locations to produce modified surface coordinate locations and wherein storing said surface coordinate locations in said computer memory comprises storing said modified surface coordinate locations in said computer memory.

4. The method of claim **1** wherein identifying said plurality of spaced apart planes comprises identifying a plurality of plane coordinate frames (222,224,226) respectively identifying locations and orientations of said plurality spaced apart planes in a three-dimensional coordinate system (314).

5. The method of claim **4** wherein identifying said plurality of plane coordinate frames comprises:

   identifying origin coordinate locations (294,296, 298) of a plurality of spaced apart points along a reference curve (288), said reference curve having a shape that generally corresponds to a shape of the three-dimensional surface in a first general direction along the surface; and
   for each respective origin coordinate location, generating data defining a plane coordinate frame having an origin located at said origin coordinate location and being oriented to cause a corresponding plane defined by said plane coordinate frame to be orthogonally oriented with respect to a portion of said reference curve passing through said origin coordinate location.

6. The method of claim **5** further comprising:

   displaying a representation (286) of said three-dimensional surface; and
   receiving operator input identifying control point locations defining said reference curve.

7. The method of claim **4** wherein identifying said plurality of plane coordinate frames comprises, for each of said plurality of plane coordinate frames, generating a modeling matrix having:

elements defining orthogonal unit vectors, said orthogonal unit vectors identifying an orientation of said plane coordinate frame; and
elements defining an origin coordinate location of said plane coordinate frame.

8. The method of claim **7** wherein determining said surface coordinate locations comprises:

determining two-dimensional surface coordinate locations with respect to said plane coordinate frame; and
transforming said two-dimensional surface coordinates into three-dimensional coordinates in said three-dimensional coordinate system using said modeling matrix.

9. The method of claim **1** further comprising:

defining at least one basis curve located on a basis curve plane intersecting the three-dimensional surface;
subdividing said at least one basis curve to identify a plurality of points along said basis curve; and
wherein identifying said plurality of basis points on each of said planes comprises projecting respective points in said plurality of points along said basis curve onto each of said planes.

10. The method of claim **9** wherein projecting said respective points comprises at least one of:

interpolating between points located on at least two basis curves located on respective basis curve planes; and
extrapolating from at least one point located on said at least one basis curve.

11. The method of claim **9** wherein defining said at least one basis curve located on said basis curve plane comprises defining at least one basis curve located on one of said spaced apart plurality of planes.

12. The method of claim **9** wherein defining said basis curve comprises defining a B-spline curve located in said plane.

13. The method of claim **1** further comprising determining two-dimensional coordinate locations on said plane of a polyline (234) linking points of intersection between said three-dimensional surface and said plane and wherein determining said offset from each said basis point comprises determining a distance between said basis point and a point of intersection between said polyline and a ray (238) extending from said basis point in a direction normal to said curve.

14. The method of claim **13** wherein determining said two-dimensional coordinate locations on said plane of said polyline comprises determining three-dimensional coordinates of said points of intersection and transforming said points of intersection into two-dimensional coordinates on said plane.

15. A computer readable medium encoded with codes for directing a processor circuit to execute the method of any one of claims **1** to **14.**

16. An apparatus for producing a computer representation of a three-dimensional surface (220,227) of an appliance (120) for a living body, the apparatus comprising means for identifying a plurality of spaced apart planes (222,224,226) intersecting the three-dimensional surface, the apparatus **characterized by**:

means for identifying a plurality of basis points (230,346,380) for each plane in said plurality of spaced apart planes, said basis points lying generally along a curve (232,334,352,358,382) on said plane;
means for determining surface coordinate locations (236) of a plurality of points on each said plane that lie on said three-dimensional surface, each surface coordinate location being defined as an offset (238,384) from one of said plurality of basis points; and
means for storing said surface coordinate locations in a computer memory (148).

17. The apparatus of claim 16 further comprising means for transforming said surface coordinate locations into a set of instructions operable to control a computer aided manufacturing machine (106) to produce the appliance.

18. The apparatus of claim 16 further comprising means for applying a shape transformation to said surface coordinate locations to produce modified surface coordinate locations and wherein said means for storing said surface coordinate locations in said computer memory comprises means for storing said modified surface coordinate locations in said computer memory.

19. The apparatus of claim 16 wherein said means for identifying said plurality of spaced apart planes comprises means for identifying a plurality of plane coordinate frames (222,224,226) respectively identifying locations and orientations of said plurality spaced apart planes in a three-dimensional coordinate system (314).

20. The apparatus of claim 19 wherein said means for identifying said plurality of plane coordinate frames comprises:

means for identifying origin coordinate locations (294,296, 298) of a plurality of spaced apart points along a reference curve (288), said reference curve having a shape that generally corresponds to a shape of the three-dimensional surface in a first general direction along the surface; and
means for generating data defining respective plane coordinate frames each having an origin located at respective origin coordinate locations and being oriented to cause a corresponding plane defined by said plane coordinate frame to be orthogonally oriented with respect to a portion of said reference curve passing through said origin coordinate location.

21. The apparatus of claim 20 further comprising:

means for displaying a representation (286) of said three-dimensional surface; and
means for receiving operator input identifying control point locations defining said reference curve.

22. The apparatus of claim 19 wherein said means for identifying said plurality of plane coordinate frames comprises means for generating a modeling matrix for each of said plurality of plane coordinate frames, each said modeling matrix having:

elements defining orthogonal unit vectors, said orthogonal unit vectors identifying an orientation of said plane coordinate frame; and
elements defining an origin coordinate location of said plane coordinate frame.

23. The apparatus of claim 22 wherein said means for determining said surface coordinate locations comprises:

means for determining two-dimensional surface coordinate locations with respect to said plane coordinate frame; and
means for transforming said two-dimensional surface coordinates into three-dimensional coordinates in said three-dimensional coordinate system using said modeling matrix.

24. The apparatus of claim 16 further comprising:

means for defining at least one basis curve located on a basis curve plane intersecting the three-dimensional surface;
means for subdividing said at least one basis curve to identify a plurality of points along said basis curve; and
wherein said means for identifying said plurality of basis points on each of said planes comprises means for projecting respective points in said plurality of points along said basis curve onto each of said planes.

25. The apparatus of claim 24
wherein said means for projecting said respective points comprises at least one of:
means for interpolating between points located on at least two basis curves located on respective basis curve planes; and
means for extrapolating from at least one point located on said at least one basis curve.

26. The apparatus of claim 24 wherein said means for defining said at least one basis curve located on said basis curve plane comprises means for defining at least one basis curve located on one of said spaced apart plurality of planes.

27. The apparatus of claim 24 wherein said means for defining said basis curve comprises means for defining a B-spline curve located in said plane.

28. The apparatus of claim 16 further comprising means for determining two-dimensional coordinate locations on said plane of a polyline (234) linking points of intersection between said three-dimensional surface and said plane and wherein said means for determining said offset from each said basis point comprises means for determining a distance between said basis point and a point of intersection between said polyline and a ray (238) extending from said basis point in a direction normal to said curve.

29. The apparatus of claim **29** wherein said means for determining said two-dimensional coordinate locations on said plane of said polyline comprises means for determining three-dimensional coordinates of said points of intersection and means for transforming said points of intersection into two-dimensional coordinates on said plane.

## Patentansprüche

1. Verfahren zum Erstellen einer Computerrepräsentation einer dreidimensionalen Oberfläche (220, 227) eines Geräts (120) für einen lebenden Körper, wobei das Verfahren das Kennzeichnen von mehreren beabstandeten Ebenen (222, 224, 226) umfasst, die die dreidimensionale Oberfläche schneiden, wobei das Verfahren **gekennzeichnet ist durch**:

   für jede Ebene in den mehreren beabstandeten Ebenen:

   Kennzeichnen von mehreren Basispunkten (230, 346, 380) auf der Ebene, wobei die Basispunkte im Allgemeinen entlang einer Kurve (232, 334, 352, 358, 382) auf der Ebene liegen;
   Bestimmen von Oberflächenkoordinatenorten (236) von mehreren Punkten auf der Ebene, die auf der dreidimensionalen Oberfläche liegen, wobei jeder Oberflächenkoordinatenort als ein Versatz (238, 384) von einem der mehreren Basispunkte definiert ist; und
   Speichern der Oberflächenkoordinatenorte in einem Computerspeicher (148).

2. Verfahren nach Anspruch 1, ferner umfassend das Umwandeln der Oberflächenkoordinatenorte in eine Gruppe von Anweisungen, die durchgeführt werden können, um eine computergestützte Produktionsmaschine (106) zum Produzieren des Geräts zu steuern.

3. Verfahren nach Anspruch 1, ferner umfassend das Anwenden einer Formumwandlung an den Oberflächenkoordinatenorten, um abgeänderte Oberflächenkoordinatenorte zu erstellen, und wobei das Speichern der Oberflächenkoordinatenorte in dem Computerspeicher das Speichern der abgeänderten Oberflächenkoordinatenorte in dem Computerspeicher umfasst.

4. Verfahren nach Anspruch 1, wobei das Kennzeichnen der mehreren beabstandeten Ebenen das Kennzeichnen von mehreren Ebenenkoordinatenrahmen (222, 224, 226) umfasst, die jeweils Orte und Ausrichtungen der mehreren beabstandeten Ebenen in einem dreidimensionalen Koordinatsystem (314) kennzeichnen.

5. Verfahren nach Anspruch 4, wobei das Kennzeichnen der mehreren Ebenenkoordinatenrahmen Folgendes umfasst:

   Kennzeichnen der Ursprungskoordinatenorte (294, 296, 298) von mehreren beabstandeten Punkten entlang einer Bezugskurve (288), wobei die Bezugskurve eine Form hat, die im Allgemeinen einer Form der dreidimensionalen Oberfläche in einer ersten allgemeinen Richtung entlang der Oberfläche entspricht; und
   für jeden jeweiligen Ursprungskoordinatenort, Erzeugen von Daten, die einen Ebenenkoordinatenrahmen definieren, der einen Ursprung hat, der an dem Ursprungskoordinatenort liegt, und ausgerichtet sind, um eine entsprechende Ebene, die durch den Ebenenkoordinatenrahmen definiert ist, dazu zu bringen, senkrecht bezüglich eines Abschnitts der Bezugskurve, die durch den Ursprungskoordinatenort hindurch verläuft, ausgerichtet zu sein.

6. Verfahren nach Anspruch 5, ferner umfassend;
   Anzeigen einer Repräsentation (286) der dreidimensionalen Oberfläche; und
   Empfangen einer Bedienereingabe, die Steuerpunktorte kennzeichnet, die die Bezugskurve definieren.

7. Verfahren nach Anspruch 4, wobei das Kennzeichnen der mehreren Ebenenkoordinatenrahmen für jeden der mehreren Ebenenkoordinatenrahmen das Erzeugen einer Modelliermatrix umfasst, die Folgendes aufweist:

   Elemente, die senkrechte Einheitsvektoren definieren, wobei die senkrechten Einheitsvektoren eine Ausrichtung des Ebenenkoordinatenrahmens kennzeichnen; und
Elemente, die einen Ursprungskoordinatenort des Ebenenkoordinatenrahmens definieren.

8. Verfahren nach Anspruch 7, wobei das Bestimmen der Oberflächenkoordinatenorte Folgendes umfasst:

Bestimmen zweidimensionaler Oberflächenkoordinatenorte bezüglich des Ebenenkoordinatenrahmens; und
Umwandeln der zweidimensionalen Oberflächenkoordinaten in dreidimensionale Koordinaten in dem dreidimensionalen Koordinatensystem unter Verwendung der Modelliermatrix.

9. Verfahren nach Anspruch 1, ferner umfassend:

Definieren mindestens einer Basiskurve, die auf einer Basiskurvenebene liegt, die die dreidimensionale Oberfläche schneidet;
Unterteilen der mindestens einen Basiskurve, um mehrere Punkte entlang der Basiskurve zu kennzeichnen; und
wobei das Kennzeichnen der mehreren Basispunkte auf jeder der Ebenen das Projizieren von jeweiligen Punkten in den mehreren Punkten entlang der Basiskurve auf jede der Ebenen umfasst.

10. Verfahren nach Anspruch 9, wobei das Projizieren der jeweiligen Punkte mindestens einen der folgenden Schritte umfasst:

Interpolieren zwischen Punkten, die auf mindestens zwei Basiskurven liegen, die an jeweiligen Basiskurvenebenen liegen; und
Extrapolieren von mindestens einem Punkt, der auf der mindestens einen Basiskurve liegt.

11. Verfahren nach Anspruch 9, wobei das Definieren der mindestens einen Basiskurve, die auf der Basiskurvenebene liegt, das Definieren mindestens einer Basiskurve, die auf einer der beabstandeten mehreren Ebenen liegt, umfasst.

12. Verfahren nach Anspruch 9, wobei das Definieren der Basiskurve das Definieren einer B-Spline-Kurve, die in der Ebene liegt, umfasst.

13. Verfahren nach Anspruch 1, ferner umfassend das Bestimmen zweidimensionaler Koordinatenorte auf der Ebene einer Polylinie (234), die Schnittpunkte zwischen der dreidimensionalen Oberfläche und der Ebene verknüpft, und wobei das Bestimmen des Versatzes von jedem Basispunkt das Bestimmen eines Abstands zwischen dem Basispunkt und einem Schnittpunkt zwischen der Polylinie und einem Strahl (238), der sich von dem Basispunkt in einer zur Kurve senkrechten Richtung erstreckt, umfasst.

14. Verfahren nach Anspruch 13, wobei das Bestimmen der zweidimensionalen Koordinatenorte auf der Ebene der Polylinie das Bestimmen dreidimensionaler Koordinaten der Schnittpunkte und das Umwandeln der Schnittpunkte in zweidimensionale Koordinaten auf der Ebene umfasst.

15. Computerlesbares Medium, das mit Codes zum Steuern einer Prozessorschaltung zum Ausführen des Verfahrens nach einem der Ansprüche 1 bis 14 codiert ist.

16. Vorrichtung zum Erstellen einer Computerrepräsentation einer dreidimensionalen Oberfläche (220, 227) eines Geräts (120) für einen lebenden Körper, wobei die Vorrichtung ein Mittel zum Kennzeichnen von mehreren beabstandeten Ebenen (222, 224, 226), die die dreidimensionale Oberfläche schneiden, umfasst, wobei die Vorrichtung durch Folgendes gekennzeichnet ist:

ein Mittel zum Kennzeichnen von mehreren Basispunkten (230, 346, 380) für jede Ebene in den mehreren beabstandeten Ebenen, wobei die Basispunkte im Allgemeinen entlang einer Kurve (232, 334, 352, 358, 382) auf der Ebene liegen;
ein Mittel zum Bestimmen von Oberflächenkoordinatenorten (236) von mehreren Punkten auf jeder der Ebenen, die auf der dreidimensionalen Oberfläche liegt, wobei jeder Oberflächenkoordinatenort als ein Versatz (238, 384) von einem der mehreren Basispunkte definiert ist; und
ein Mittel zum Speichern der Oberflächenkoordinatenorte in einem Computerspeicher (148).

17. Vorrichtung nach Anspruch 16, ferner umfassend ein Mittel zum Umwandeln der Oberflächenkoordinatenorte in eine Gruppe von Anweisungen, die durchgeführt werden können, um eine computergestützte Produktionsmaschine (106) zum Produzieren des Geräts zu steuern.

**18.** Vorrichtung nach Anspruch 16, ferner umfassend ein Mittel zum Anwenden einer Formumwandlung an den Oberflächenkoordinatenorten, um abgeänderte Oberflächenkoordinatenorte zu erstellen, und wobei das Mittel zum Speichern der Oberflächenkoordinatenorte in dem Computerspeicher ein Mittel zum Speichern der abgeänderten Oberflächenkoordinatenorte in dem Computerspeicher umfasst.

**19.** Vorrichtung nach Anspruch 16, wobei das Mittel zum Kennzeichnen der mehreren beabstandeten Ebenen ein Mittel zum Kennzeichnen von mehreren Ebenenkoordinatenrahmen (222, 224, 226) umfasst, die jeweils Orte und Ausrichtungen der mehreren beabstandeten Ebenen in einem dreidimensionalen Koordinatensystem (314) kennzeichnen.

**20.** Vorrichtung nach Anspruch 19, wobei das Mittel zum Kennzeichnen der mehreren Ebenenkoordinatenrahmen Folgendes umfasst:

ein Mittel zum Kennzeichnen von Ursprungskoordinatenorten (294, 296, 298) von mehreren beabstandeten Punkten entlang einer Bezugskurve (288), wobei die Bezugskurve eine Form hat, die im Allgemeinen einer Form der dreidimensionalen Oberfläche in einer ersten allgemeinen Richtung entlang der Oberfläche entspricht; und
ein Mittel zum Erzeugen von Daten, die jeweilige Ebenenkoordinatenrahmen definieren, die je einen Ursprung haben, der an jeweiligen Ursprungskoordinatenorten liegt, und ausgerichtet sind, um eine entsprechende Ebene, die durch den Ebenenkoordinatenrahmen definiert ist, dazu zu bringen, senkrecht bezüglich eines Abschnitts der Bezugskurve, die durch den Ursprungskoordinatenort hindurch verläuft, ausgerichtet zu sein.

**21.** Vorrichtung nach Anspruch 20, ferner umfassend:

ein Mittel zum Anzeigen einer Repräsentation (286) der dreidimensionalen Oberfläche; und
ein Mittel zum Empfangen einer Bedienereingabe, die Steuerpunktorte kennzeichnet, die die Bezugskurve definieren.

**22.** Vorrichtung nach Anspruch 19, wobei das Mittel zum Kennzeichnen der mehreren Ebenenkoordinatenrahmen ein Mittel zum Erzeugen einer Modelliermatrix für jeden der mehreren Ebenenkoordinatenrahmen umfasst, wobei jede der Modelliermatrizen Folgendes aufweist:

Elemente, die senkrechte Einheitsvektoren definieren, wobei die senkrechten Einheitsvektoren eine Ausrichtung des Ebenenkoordinatenrahmens kennzeichnen; und
Elemente, die einen Ursprungskoordinatenort des Ebenenkoordinatenrahmens definieren.

**23.** Vorrichtung nach Anspruch 22, wobei das Mittel zum Bestimmen der Oberflächenkoordinatenorte Folgendes umfasst:

ein Mittel zum Bestimmen zweidimensionaler Oberflächenkoordinatenorte bezüglich des Ebenenkoordinatenrahmens; und
ein Mittel zum Umwandeln der zweidimensionalen Oberflächenkoordinaten in dreidimensionale Koordinaten in dem dreidimensionalen Koordinatensystem unter Verwendung der Modelliermatrix.

**24.** Vorrichtung nach Anspruch 16, ferner umfassend:

ein Mittel zum Definieren mindestens einer Basiskurve, die auf einer Basiskurvenebene liegt, die die dreidimensionale Oberfläche schneidet;
ein Mittel zum Unterteilen der mindestens einen Basiskurve, um mehrere Punkte entlang der Basiskurve zu kennzeichnen; und
wobei das Mittel zum Kennzeichnen der mehreren Basispunkte auf jeder der Ebenen ein Mittel zum Projizieren von jeweiligen Punkten in den mehreren Punkten entlang der Basiskurve auf jede der Ebenen umfasst.

**25.** Vorrichtung nach Anspruch 24, wobei das Mittel zum Projizieren der jeweiligen Punkte mindestens eines der Folgenden umfasst:

ein Mittel zum Interpolieren zwischen Punkten, die auf mindestens zwei Basiskurven liegen, die auf jeweiligen Basiskurvenebenen liegen; und
ein Mittel zum Extrapolieren von mindestens einem Punkt, der auf der mindestens einen Basiskurve liegt.

26. Vorrichtung nach Anspruch 24, wobei das Mittel zum Definieren der mindestens einen Basiskurve, die auf der Basiskurvenebene liegt, ein Mittel zum Definieren mindestens einer Basiskurve, die auf einer der beabstandeten mehreren Ebenen liegt, umfasst.

27. Vorrichtung nach Anspruch 24, wobei das Mittel zum Definieren der Basiskurve ein Mittel zum Definieren einer B-Spline-Kurve, die in der Ebene liegt, umfasst.

28. Vorrichtung nach Anspruch 16, ferner umfassend ein Mittel zum Bestimmen zweidimensionaler Koordinatenorte auf der Ebene einer Polylinie (234), die Schnittpunkte zwischen der dreidimensionalen Oberfläche und der Ebene verknüpft, und wobei das Mittel zum Bestimmen des Versatzes von jedem der Basispunkte ein Mittel zum Bestimmen eines Abstands zwischen dem Basispunkt und einem Schnittpunkt zwischen der Polylinie und einem Strahl (238), der sich von dem Basispunkt in einer zur Kurve senkrechten Richtung erstreckt, umfasst.

29. Vorrichtung nach Anspruch 29, wobei das Mittel zum Bestimmen der zweidimensionalen Koordinatenorte auf der Ebene der Polylinie ein Mittel zum Bestimmen dreidimensionaler Koordinaten der Schnittpunkte und ein Mittel zum Umwandeln der Schnittpunkte in zweidimensionale Koordinaten auf der Ebene umfasst.

## Revendications

1. Procédé pour produire une représentation par ordinateur d'une surface tridimensionnelle (220, 227) d'un appareil (120) pour un corps humain, le procédé comprenant l'identification d'une pluralité de plans (222, 224, 226) espacés coupant la surface tridimensionnelle, le procédé **se caractérisant par** :

   pour chaque plan de ladite pluralité de plans espacés :

   l'identification d'une pluralité de points de base (230, 346, 380) sur ledit plan, lesdits points de base se situant généralement le long d'une courbe (232, 334, 352, 358, 382) sur ledit plan ;
   la détermination de positions de coordonnées de surface (236) d'une pluralité de points sur ledit plan qui se situent sur ladite surface tridimensionnelle, chaque position de coordonnées de surface étant définie en tant que décalage (238, 384) par rapport à l'un de ladite pluralité de points de base ; et
   le stockage desdites positions de coordonnées de surface dans une mémoire (148) d'ordinateur.

2. Procédé selon la revendication 1, comprenant par ailleurs la transformation desdites positions de coordonnées de surface en un ensemble d'instructions exploitables pour contrôler une machine de fabrication assistée par ordinateur (106) pour produire l'appareil.

3. Procédé selon la revendication 1, comprenant par ailleurs l'application d'une transformation de forme auxdites positions de coordonnées de surface pour produire des position de coordonnées de surface modifiées, et dans lequel le stockage desdites positions de coordonnées de surface dans ladite mémoire d'ordinateur comprend le stockage desdites positions de coordonnées de surface modifiées dans ladite mémoire d'ordinateur.

4. Procédé selon la revendication 1, dans lequel l'identification de ladite pluralité de plans espacés comprend l'identification d'une pluralité de fenêtres de coordonnées (222, 224, 226) de plan identifiant respectivement des positions et orientations de ladite pluralité de plans espacés dans un système de coordonnées tridimensionnel (314).

5. Procédé selon la revendication 4, dans lequel l'identification de ladite pluralité de fenêtres de coordonnées de plan comprend :

   l'identification de positions de coordonnées d'origine (294, 296, 298) d'une pluralité de points espacés le long d'une courbe de référence (288), ladite courbe de référence ayant une forme qui correspond généralement à une forme de la surface tridimensionnelle dans une première direction générale le long de la surface ; et
   pour chaque position de coordonnées d'origine respective, la génération de données définissant une fenêtre de coordonnées de plan ayant une origine située dans ladite position de coordonnées d'origine et orientée de manière à faire en sorte qu'un plan correspondant défini par ladite fenêtre de coordonnées de plan soit orienté orthogonalement par rapport à une partie de ladite courbe de référence passant par ladite position de coordonnées d'origine.

6.  Procédé selon la revendication 5, comprenant :

l'affichage d'une représentation (286) de ladite surface tridimensionnelle ; et
la réception d'une saisie d'opérateur identifiant des positions de points de contrôle définissant ladite courbe de référence.

7.  Procédé selon la revendication 4, dans lequel l'identification de ladite pluralité de fenêtres de coordonnées de plan comprend, pour chacune de ladite pluralité de fenêtres de coordonnées de plan, la génération d'une matrice de modélisation ayant :

des éléments définissant des vecteurs unitaires orthogonaux, lesdits vecteurs unitaires orthogonaux identifiant une orientation de ladite fenêtre de coordonnées de plan ; et
des éléments définissant une position de coordonnées d'origine de ladite fenêtre de coordonnées de plan.

8.  Procédé selon la revendication 7, dans lequel la détermination desdites positions de coordonnées de surface comprend :

la détermination de positions de coordonnées de surface bidimensionnelles par rapport à ladite fenêtre de coordonnées de plan ; et
la transformation desdites coordonnées de surface bidimensionnelles en coordonnées tridimensionnelles dans ledit système de coordonnées tridimensionnelles en utilisant ladite matrice de modélisation.

9.  Procédé selon la revendication 1, comprenant par ailleurs :

la définition d'au moins une courbe de base située sur un plan de courbe de base coupant la surface tridimensionnelle ;
la subdivision de ladite au moins une courbe de base pour identifier une pluralité de points le long de ladite courbe de base ; et
dans lequel l'identification de ladite pluralité de points de base sur chacun desdits plans comprend la projection de points respectifs de ladite pluralité de points le long de ladite courbe de base sur chacun desdits plans.

10. Procédé selon la revendication 9, dans lequel la projection desdits points respectifs comprend au moins l'une d'entre :

une interpolation entre des points situés sur au moins deux courbes de base situées sur des plans respectifs de courbes de base ; et
une extrapolation à partir d'au moins un point situé sur ladite au moins une courbe de base.

11. Procédé selon la revendication 9, dans lequel la définition de ladite au moins une courbe de base située sur ledit plan de courbe de base comprend la définition d'au moins une courbe de base située sur l'un de ladite pluralité de plans espacés.

12. Procédé selon la revendication 9, dans lequel la définition de ladite courbe de base comprend la définition d'une courbe B-spline située dans ledit plan.

13. Procédé selon la revendication 1, comprenant en outre la détermination de positions de coordonnées bidimensionnelles sur ledit plan d'une polyligne (234) reliant des points d'intersection entre ladite surface tridimensionnelle et ledit plan, et dans lequel la détermination dudit décalage par rapport à chacun desdits points de base comprend la détermination d'une distance entre ledit point de base et un point d'intersection entre ladite polyligne et un rayon (238) s'étendant à partir dudit point de base dans une direction normale à ladite courbe.

14. Procédé selon la revendication 13, dans lequel la détermination desdites positions de coordonnées bidimensionnelles sur ledit plan de ladite polyligne comprend la détermination de coordonnées tridimensionnelles desdits points d'intersection et la transformation desdits points d'intersection en coordonnées bidimensionnelles sur ledit plan.

**15.** Support lisible par informatique codé avec des codes pour inciter un circuit de processeur à exécuter le procédé selon l'une quelconque des revendications 1 à 14.

**16.** Appareil pour produire une représentation par ordinateur d'une surface tridimensionnelle (220, 227) d'un appareil (120) pour un corps humain, l'appareil comprenant un moyen pour identifier une pluralité de plans (222, 224, 226) espacés coupant la surface tridimensionnelle, l'appareil **se caractérisant par** :

un moyen pour identifier une pluralité de points de base (230, 346, 380) pour chaque plan de ladite pluralité de plans espacés, lesdits points de base se situant généralement le long d'une courbe (232, 334, 352, 358, 382) sur ledit plan ;
un moyen pour déterminer des positions de coordonnées de surface (236) d'une pluralité de points sur chacun desdits plan qui se situent sur ladite surface tridimensionnelle, chaque position de coordonnées de surface étant définie en tant que décalage (238, 384) par rapport à l'un de ladite pluralité de points de base ; et
un moyen pour stocker lesdites positions de coordonnées de surface dans une mémoire d'ordinateur (148).

**17.** Appareil selon la revendication 16,
comprenant par ailleurs un moyen pour transformer lesdites positions de coordonnées de surface en un ensemble d'instructions exploitables pour contrôler une machine de fabrication assistée par ordinateur (106) afin de produire l'appareil.

**18.** Appareil selon la revendication 16,
comprenant par ailleurs un moyen pour appliquer une transformation de forme auxdites positions de coordonnées de surface pour produire des positions de coordonnées de surface modifiées, et dans lequel ledit moyen pour stocker lesdites positions de coordonnées de surface dans ladite mémoire d'ordinateur comprend un moyen pour stocker lesdites positions de coordonnées de surface modifiées dans ladite mémoire d'ordinateur.

**19.** Appareil selon la revendication 16,
dans lequel ledit moyen pour identifier ladite pluralité de plans espacés comprend un moyen pour identifier une pluralité de fenêtres de coordonnées de plan (222, 224, 226) identifiant respectivement des positions et orientations de ladite pluralité de plans espacés dans un système de coordonnées tridimensionnelles (314).

**20.** Appareil selon la revendication 19, dans lequel ledit moyen pour identifier ladite pluralité de fenêtres de coordonnées de plan comprend :

un moyen pour identifier des positions de coordonnées d'origine (294, 296, 298) d'une pluralité de points espacés le long d'une courbe de référence (288), ladite courbe de référence ayant une forme qui correspond généralement à une forme de la surface tridimensionnelle dans une première direction générale le long de la surface ; et
un moyen pour générer des données définissant des fenêtres de coordonnées de plan respectives ayant chacune une origine située dans des positions de coordonnées d'origine et orientée de manière à faire en sorte qu'un plan correspondant défini par ladite fenêtre de coordonnées de plan soit orienté orthogonalement par rapport à une partie de ladite courbe de référence passant par ladite position de coordonnées d'origine.

**21.** Appareil selon la revendication 20, comprenant par ailleurs :

un moyen pour afficher une représentation (286) de ladite surface tridimensionnelle ; et
un moyen pour recevoir une saisie opérateur identifiant des positions de points de contrôle définissant ladite courbe de référence.

**22.** Appareil selon la revendication 19, dans lequel ledit moyen pour identifier ladite pluralité de fenêtres de coordonnées de plan comprend un moyen pour générer une matrice de modélisation pour chacune de ladite pluralité de fenêtres de coordonnées de plan, chacune desdites matrices de modélisation ayant :

des éléments définissant des vecteurs unitaires orthogonaux, lesdits vecteurs unitaires orthogonaux identifiant une orientation de ladite fenêtre de coordonnées de plan ; et
des éléments définissant une position de coordonnées d'origine de ladite fenêtre de coordonnées de plan.

**23.** Appareil selon la revendication 22, dans lequel ledit moyen pour déterminer lesdites positions de coordonnées de surface comprend :

un moyen pour déterminer des positions de coordonnées de surface bidimensionnelles par rapport à ladite fenêtre de coordonnées de plan ; et

un moyen pour transformer lesdites coordonnées de surface bidimensionnelles en coordonnées tridimensionnelles dans ledit système de coordonnées tridimensionnelles en utilisant ladite matrice de modélisation.

**24.** Appareil selon la revendication 16, comprenant par ailleurs :

un moyen pour définir au moins une courbe de base située sur un plan de courbe de base coupant la surface tridimensionnelle ;

un moyen pour subdiviser ladite au moins une courbe de base pour identifier une pluralité de points le long de ladite courbe de base ; et

dans lequel ledit moyen pour identifier ladite pluralité de points de base sur chacun desdits plans comprend un moyen pour projeter des points respectifs de ladite pluralité de points le long de ladite courbe de base sur chacun desdits plans.

**25.** Appareil selon la revendication 24, dans lequel ledit moyen pour projeter lesdits points respectifs comprend au moins l'un d'entre :

un moyen pour l'interpolation entre des points situés sur au moins deux courbes de base situées sur des plans de courbes de base respectifs ; et

un moyen pour l'extrapolation à partir d'au moins un point situé sur ladite au moins une courbe de base.

**26.** Appareil selon la revendication 24, dans lequel ledit moyen pour définir ladite au moins une courbe de base située sur ledit plan de courbe de base comprend un moyen pour définir au moins une courbe de base située sur l'un de ladite pluralité de plans espacés.

**27.** Appareil selon la revendication 24, dans lequel ledit moyen pour définir ladite courbe de base comprend un moyen pour définir une courbe B-spline située dans ledit plan.

**28.** Appareil selon la revendication 16, comprenant par ailleurs un moyen pour déterminer des positions de coordonnées bidimensionnelles sur ledit plan d'une polyligne (234) reliant des points d'intersection entre ladite surface tridimensionnelle et ledit plan, et dans lequel ledit moyen pour déterminer ledit décalage par rapport à chacun desdits points de base comprend un moyen pour déterminer une distance entre ledit point de base et un point d'intersection entre ladite polyligne et un rayon (238) s'étendant à partir dudit point de base dans une direction normale à ladite courbe.

**29.** Appareil selon la revendication 29, dans lequel ledit moyen pour déterminer lesdites positions de coordonnées bidimensionnelles sur ledit plan de ladite polyligne comprend un moyen pour déterminer des coordonnées tridimensionnelles desdits points d'intersection et un moyen pour transformer lesdits points d'intersection en coordonnées bidimensionnelles sur ledit plan.

FIG. 1

FIG. 2

200

Receive input plurality of
coordinates
202

Identify plurality of spaced
apart planes intersecting the
three-dimensional surface
204

Select the first plane for
processing
205

Identify a plurality of basis
points on the plane
206

Determine surface coordinate
locations of points on the plane
208

Store the surface coordinate locations
of the points in computer memory
210

Next
Plane
214

Last
plane
processed?
212

N

Y

216

End

FIG. 3

FIG. 4

FIG. 5

FIG. 6

260

```
┌─────────────────────────┐
│   Receive operator input │
│ identifying reference curve │
│           262           │
└─────────────────────────┘
            │
            ▼
┌─────────────────────────┐
│  Identify origin coordinate │
│     locations along the    │
│       reference curve       │
│           264           │
└─────────────────────────┘
            │
            ▼
┌─────────────────────────┐
│   Select the first plane for │
│        processing           │
│           265           │
└─────────────────────────┘
            │
            ▼
┌─────────────────────────┐
│ Generate data defining plane │      ┌──────────┐
│  coordinate frame at each   │◄─────│  Next    │
│   origin coordinate location │      │  Plane   │
│           266           │      │   274    │
└─────────────────────────┘      └──────────┘
            │                         ▲
            ▼                         │
┌─────────────────────────┐          │
│   Store coordinate frame   │          │
│     data in memory         │          │
│           268           │          │
└─────────────────────────┘          │
            │                         │
            ▼                         │
          ╱╲                          │
         ╱  ╲                         │
        ╱ Last ╲        N             │
       ╱ plane? ╲───────────────────┘
       ╲  270   ╱
        ╲      ╱
         ╲    ╱
          ╲  ╱
           Y
           │        ╭─272
           ▼
          End
```

FIG. 7

FIG. 8

FIG. 9

320

Receive operator input of
basis curve plane location ——322

Receive operator input
identifying basis curve on the
basis plane
324

Subdivide basis curve
to identify points along
the basis curve
326

Project the points onto each of
the identified planes to identify
basis points
328

Save basis point data in
memory
329

FIG. 10

FIG. 11

FIG. 12

356

**FIG. 13**

FIG. 14

400

Determine polyline intersection of
the three-dimensional shape with
the plane in (x,y,z) coordinates
402

Transform the polyline
intersection into two-dimensional
*uv* plane coordinates
404

Determine intersection of the
basis point unit vectors with
the polyline
406

Store in memory as
surface coordinates for
the plane
408

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003299679 B